# EUROPÄISCHE PATENTANMELDUNG

(11) **EP 1 894 536 A1**
(43) Veröffentlichungstag der Anmeldung: **05.03.2008**
(21) Anmeldenummer: 07075904.8
(22) Anmeldetag: 19.08.2004
(51) Int. Cl.: A61B 18/14, A61B 5/042, A61B 5/055, A61B 6/03, A61B 8/14, G01S 15/89

(54) **Verfahren und Vorrichtung zur visuellen Unterstützung einer elektrophysiologischen Katheteranwendung im Herzen**

(30) Priorität: 01.09.2003 DE 10340544
(62) Teilanmeldung aus: 04764298.8
(71) Anmelder: Biosense Webster, Inc., Diamond Bar, CA 91765 (US)
(72) Erfinder: Sauer, Frank, Princeton, New Jersey 08540 (US); Xu, Chenyang, Allentown, New Jersey 08501 (US); Fuimaono, Kristine, West Berlin, New Jersey 08091 (US); Hayam, Gal, 36501 Tivon (IL); Karmi, Yuval, 38386 Hadera (IL); Killmann, Reinmar, 91301 Forchheim (DE); Preiss, Assaf, 17906 Shimshit (IL); Rahn, Norbert, 91301 Forchheim (DE)
(74) Vertreter: Small, Gary James

(57) **Zusammenfassung**

Die vorliegende Erfindung betrifft ein Verfahren und eine Vorrichtung zur visuellen Unterstützung einer elektrophysiologischen Katheteranwendung im Herzen, bei denen während der Durchführung der Katheteranwendung bereitgestellte elektroanatomische 3D-Mapping-Daten eines zu behandelnden Bereiches des Herzens visualisiert warden. Vor der Durchführung der Katheteranwendung warden mit einem Verfahren der tomographischen 3D-Bildgebung 3D-Bilddaten des zu behandelnden Bereiches erfasst, aus den 3D-Bilddaten durch Segmentierung ein 3D-Oberflächenverlauf von Objekten in dem zu behandelnden Bereich extrahiert und anschließend die bereitgestellten elektroanatomischen 3D-Mapping-Daten und den 3D-Oberflächenverlauf bildende 3D-Bilddaten lage- und dimensionsrichtig zugeordnet und z.B. während der Durchführung der Katheteranwendung einander überlagert visualisiert. Das vorliegende Verfahren und die zugehörige Vorrichtung ermöglichen eine verbesserte Orientierung des Bedieners bei der Durchführung einer elektrophysiologischen Katheteranwendung am Herzen.

## Beschreibung

Die vorliegende Erfindung betrifft ein Verfahren und eine Vorrichtung zur visuellen Unterstützung einer elektrophysiologischen Katheteranwendung im Herzen, bei denen während der Durchführung der Katheteranwendung bereitgestellte elektroanatomische 3D-Mapping-Daten eines zu behandelnden Bereiches des Herzens visualisiert werden.

Die Behandlung von Herzrhythmus-Störungen hat sich seit der Einführung der Technik der Katheterablation mittels Hochfrequenzstrom wesentlich gewandelt. Bei dieser Technik wird unter Röntgenkontrolle ein Ablations-Katheter über Venen oder Arterien in eine der Herzkammern eingeführt und durch Hochfrequenzstrom das die Herzrhythmus-Störungen hervorrufende Gewebe verödet. Voraussetzung für eine erfolgreiche Durchführung einer Katheterablation ist die genaue Ortung der Ursache der Herzrhythmus-Störung in der Herzkammer. Diese Ortung erfolgt über eine elektrophysiologische Untersuchung, bei der elektrische Potentiale mit einem in die Herzkammer eingeführten Mapping-Katheter ortsaufgelöst erfasst werden. Aus dieser elektrophysiologischen Untersuchung, dem so genannten elektroanatomischen Mapping, werden somit 3D-Mapping-Daten erhalten, die an einem Monitor visualisiert werden können. Die Mapping-Funktion und die Ablations-Funktion sind dabei in vielen Fällen in einem Katheter vereint, so dass der Mapping-Katheter gleichzeitig auch ein Ablations-Katheter ist.

Ein bekanntes elektroanatomisches 3D-Mapping-Verfahren, wie es mit dem Carto-System der Fa. Biosense Webster Inc., USA, durchführbar ist, basiert auf elektromagnetischen Prinzipien. Unter dem Untersuchungstisch werden drei verschiedene magnetische Wechselfelder geringer Intensität aufgebaut. Mittels integrierter elektromagnetischer Sensoren an der Katheterspitze des Mapping-Katheters ist es möglich, die durch Katheterbewegungen induzierten Spannungsänderungen innerhalb des Magnetfeldes zu messen und mit Hilfe mathematischer Algorithmen zu jedem Zeitpunkt die Position des Mapping-Katheters zu errechnen. Durch punktweises Abtasten der endokardialen Kontur einer Herzkammer mit dem Mapping-Katheter bei simultaner Erfassung der elektrischen Signale entsteht eine elektroanatomische dreidimensionale Landkarte, in der die elektrischen Signale farbkodiert wiedergegeben werden.

Die für die Führung des Katheters erforderliche Orientierung des Bedieners erfolgt in der Regel bisher über fluoroskopische Visualisierung. Da die Position des Mapping-Katheters beim elektroanatomischen Mapping jederzeit bekannt ist, kann bei dieser Technik nach Erfassung einer genügend großen Anzahl von Messpunkten die Orientierung auch durch kontinuierliche Darstellung der Katheterspitze in der elektroanatomischen Landkarte erfolgen, so dass in diesem Stadium auf eine fluoroskopische Bildtechnik mit Röntgendurchleuchtung verzichtet werden kann.

Die bisher nicht optimalen Orientierungsmöglichkeiten des Bedieners bei der Führung des Katheters stellen ein grundsätzliches Problem bei der Durchführung der Katheterablation innerhalb des Herzens dar. Eine genauere Darstellung der morphologischen Umgebung während der Führung des Katheters würde einerseits die Genauigkeit bei der Katheterablation erhöhen und andererseits auch die Zeit für die Durchführung des elektroanatomischen Mapping verkürzen. Weiterhin könnte die in vielen Fällen für das elektroanatomische Mapping noch erforderliche Röntgendurchleuchtung verringert oder vermieden und damit auch die applizierte Röntgensdosis reduziert werden.

Zur Verbesserung der Orientierung des Bedieners bei der Führung des Katheters sind unterschiedliche Techniken bekannt. Bei einer Technik wird ein spezieller Katheter mit einem Ultraschall-Messkopf eingesetzt, wie er beispielsweise von der Fa. Siemens AG Medical Solutions unter der Bezeichnung Acunav angeboten wird. Über eine zweidimensionale Ultraschallerfassung der Umgebung sowie eines Teils des Katheters können Teile des zu verödenden Zielgewebes zusammen mit dem Katheter in Echtzeit visualisiert werden. Der Einsatz eines derartigen Katheters liefert allerdings keine dreidimensionale Bildinformation. Die Ultraschalldarstellung kann daher nur eingesetzt werden, um beispielsweise einen so genannten Lasso-Katheter in die Öffnung einer Pulmonalvene einzusetzen. Nach der Positionierung des Lasso-Katheters kann eine Gewebeverödung um die Öffnung der Pulmonalvene unter Visualisierung sowohl des Lasso-Katheters als auch des Ablations-Katheters mittels Röntgenstrahlung durchgeführt werden.

Bei einer anderen bekannten Technik wird ein Lasso-Katheter ohne Unterstützung bildgebender 2D-Ultraschalltechnik an der Öffnung der Pulmonalvene platziert, indem ein Kontrastmittel über einen im linken Vorhof im Bereich der Pulmonalvenenöffnung geführten Katheter unter Röntgendurchleuchtung appliziert wird. Das Kontrastmittel verteilt sich dabei, wobei ein kleiner Teil mit dem Blutfluss über die Pulmonalvene austritt. Diese Kurzzeit-Visualisierung der Pulmonalvene ermöglicht die Platzierung des Lasso-Katheters in der Öffnung. Die Katheterablation kann nachfolgend wie bei der vorgenannten Technik durchgeführt werden.

Weiterhin ist eine Technik bekannt, bei der die Öffnung der Pulmonalvene durch elektroanatomisches Mapping des linken Vorhofs und der Pulmonalvenen lokalisiert wird, indem der Mapping-Katheter zunächst in eine Pulmonalvene eingeführt und anschließend zurückgezogen wird, bis elektrische Aktivität des Vorhofs detektiert wird. Diese Position entspricht der Position der Öffnung der Pulmonalvene, um die das Zielgewebe verödet werden soll.

Die Aufgabe der vorliegenden Erfindung besteht darin, ein Verfahren sowie eine Vorrichtung zur visuellen Unterstützung einer elektrophysiologischen Katheteranwendung im Herzen anzugeben, die eine verbesserte Orientierung während der Führung des Katheters bei der Katheteranwendung, insbesondere beim elektroanatomischen Mapping und/oder bei einer Katheterablation ermöglichen.

Die Aufgabe wird mit dem Verfahren sowie der Vorrichtung gemäß den Patentansprüchen 1 und 16 gelöst. Vorteilhafte Ausgestaltungen des Verfahrens sowie der Vorrichtung sind Gegenstand der Unteransprüche oder lassen sich aus der nachfolgenden Beschreibung sowie den Ausführungsbeispielen entnehmen.

Bei dem vorliegenden Verfahren zur visuellen Unterstützung einer elektrophysiologischen Katheteranwendung im Herzen, insbesondere einer Katheterablation, werden vor der Durchführung der Katheteranwendung zunächst 3D-Bilddaten des zu behandelnden Bereiches mit einem Verfahren der tomographischen 3D-Bildgebung erfasst. Aus den 3D-Bilddaten wird durch Segmentierung ein 3D-Oberflächenverlauf von Objekten, insbesondere einer oder mehrerer Herzkammern oder Gefäße, in dem zu behandelnden Bereich extrahiert. Die den 3D-Oberflächenverlauf bildenden 3D-Bilddaten, im Folgenden als selektierte 3D-Bilddaten bezeichnet, werden den während der Durchführung der Katheteranwendung bereitgestellten elektroanatomischen 3D-Mapping-Daten lage- und dimensionsrichtig zugeordnet. Die 3D-Mapping-Daten und zumindest die selektierten 3D-Bilddaten werden dann, vorzugsweise während der Durchführung der Katheteranwendung, in einer visuellen Darstellung lage- und dimensionsrichtig einander überlagert visualisiert.

Durch diese Überlagerung des 3D-Oberflächenverlaufs, durch den die Morphologie des zu behandelnden bzw. des behandelten Bereiches sehr gut wiedergeben wird, mit den während der Durchführung der Katheteranwendung erfassten elektroanatomischen 3D-Mapping-Daten werden dem Bediener des Katheters bei der Durchführung der Katheteranwendung eine bessere Orientierung sowie genauere Details vermittelt als dies bei den bisher bekannten Verfahren zur visuellen Unterstützung der Fall ist. Die überlagerte Bilddarstellung kann beispielsweise an einem Monitor im Kontrollraum oder im Arbeitsraum selbst erfolgen. Auf dem Monitor erkennt der Bediener dann während der Durchführung der Katheteranwendung das anatomische Gewebe und dessen elektrophysiologische Eigenschaften innerhalb einer Darstellung in Echtzeit. Dies ermöglicht eine sichere und genaue Arbeitsweise.

Für die Erfassung der 3D-Bilddaten können beispielsweise Verfahren der Röntgen-Computer-Tomographie, der Magnetresonanz-Tomographie oder der 3D-Ultraschall-Bildgebung eingesetzt werden. Auch Kombinationen dieser Bildgebungsverfahren sind selbstverständlich möglich. Es muss lediglich darauf geachtet werden, dass die 3D-Bildaufnahmen bei der gleichen Herzphase erfolgen wie die bereitgestellten elektroanatomischen 3D-Mapping-Daten, um jeweils den gleichen Zustand des Herzens zu erfassen. Dies kann mit der bekannten Technik des EKG-Gating bei der Erfassung der Bilddaten sowie der elektroanatomischen Mapping-Daten gewährleistet werden.

Für die Segmentierung der erfassten 3D-Bilddaten lassen sich unterschiedliche Techniken einsetzen. So kann der dreidimensionale Oberflächenverlauf der in den 3D-Bilddaten enthaltenen Objekte, insbesondere der Gefäße und/oder einer oder mehrerer Herzkammern, beispielsweise durch Segmentierung aller mit dem bildgebenden Verfahren erhaltenen 2D-Schichten erfolgen. Neben dieser schichtweisen Segmentierung ist auch eine 3D-Segmentierung eines oder mehrerer Kammern und/oder Gefäße möglich. Geeignete Segmentierungstechniken sind dem Fachmann aus dem Gebiet der Bildverarbeitung medizinischer Bilddaten bekannt.

Die dimensions- und lagerichtige Zuordnung der elektroanatomischen 3D-Mapping-Daten und der selektierten 3D-Bilddaten kann durch unterschiedliche Techniken erfolgen. Eine Möglichkeit besteht in der Registrierung zwischen den jeweiligen Daten durch visuelle Anpassung des 3D-Oberflächenverlaufs an die Darstellung der elektroanatomischen 3D-Mapping-Daten. Weiterhin lassen sich auch künstliche Marker oder natürliche markante Punkte nutzen, die in beiden Datensätzen erkennbar sind. Für die Registrierung kann neben dem zu behandelnden Bereich auch ein Nachbarbereich herangezogen werden, soweit dieser in den vorhandenen Daten enthalten ist. Weiterhin ist es möglich, den Schwerpunkt bei der Durchführung der Registrierung auf Daten in der Umgebung des zu verödenden Gewebes, im Folgenden auch als Zielgewebe bezeichnet, oder der Katheterspitze zu legen. In einer vorteilhaften Ausgestaltung des Verfahrens sowie der Vorrichtung erfolgt die Registrierung in einem ersten Stadium, in dem nur ein kleinerer Teil der elektroanatomischen 3D-Mapping-Daten vorliegt, mit Hilfe von künstlichen Markern oder von markanten Punkten und in einem oder mehreren nachfolgenden Stadien, in denen bereits eine größere Anzahl elektroanatomischer 3D-Mapping-Daten vorliegt, durch Oberflächenanpassung. Auf diese Weise wird die Registrierung während der Katheteranwendung mit zunehmender Anzahl elektroanatomischer 3D-Mapping-Daten verbessert.

Bei der Überlagerung der 3D-Bilddaten mit den elektroanatomischen 3D-Mapping-Daten kann die Darstellung der 3D-Bilddaten mittels einer Volume-Rendering-Technik erfolgen. In einer weiteren Ausgestaltung wird der 3D-Oberflächenverlauf durch ein Polygonnetz dargestellt, wie dies aus dem Bereich der Computergraphik bekannt ist. Die Überlagerung kann mit einstellbarer Transparenz und einstellbarem Blendingfaktor erfolgen. Auch eine endoskopische Perspektive kann berechnet und dargestellt werden. Da die elektroanatomischen 3D-Mapping-Daten auch die jeweilige momentane Position der Katheterspitze enthalten, kann zeitweise auch lediglich die Position des Katheters in Echtzeit in der Darstellung der 3D-Bilddaten visualisiert werden, ohne die übrigen 3D-Mapping-Daten darzustellen. Weiterhin lässt sich aufgrund der Registrierung zwischen den 3D-Mapping-Daten und den 3D-Bilddaten die Entfernung des Katheters zu beliebigen Bildpunkten der 3D-Bilddaten berechnen. Dies ermöglicht eine vorteilhafte Ausgestaltung des vorliegenden Verfahrens, bei der die Katheterspitze in der Visualisierung farbig dargestellt wird, wobei sich die Farbe in Abhängigkeit von der Entfernung zu vorgebbaren Bildpunkten, insbesondere der Position des Zielgewebes, verändert.

Die vorliegende Vorrichtung zur Durchführung des Verfahrens umfasst eine oder mehrere Eingangsschnittstellen für die elektroanatomischen 3D-Mapping-Daten und die mit einem bildgebenden tomographischen Verfahren erfassten 3D-Bilddaten. Die Vorrichtung weist ein Segmentierungsmodul für die Segmentierung der 3D-Bilddaten auf, um einen 3D-oberflächenverlauf von Objekten zu extrahieren, die innerhalb des mit den 3D-Bilddaten erfassten Volumens enthalten sind. Mit diesem Segmentierungsmodul ist ein Registrierungsmodul verbunden, das für die lage- und dimensionsrichtige Zuordnung der elektroanatomischen 3D-Mapping-Daten und der den 3D-Oberflächenverlauf bildenden 3D-Bilddaten ausgebildet ist. Mit diesem Registrierungsmodul ist wiederum ein Visualisierungsmodul verbunden, das die 3D-Mapping-Daten und zumindest die den 3D-Oberflächenverlauf bildenden 3D-Bilddaten lage- und dimensionsrichtig einander überlagert zur Visualisierung mit einem Anzeigegerät, insbesondere einem Monitor oder Projektor, bereitstellt.

Die einzelnen Module der Vorrichtung sind in unterschiedlichen Ausgestaltungen entsprechend zur Durchführung der nachfolgend aufgezeigten unterschiedlichen Ausführungsformen des Verfahrens ausgebildet.

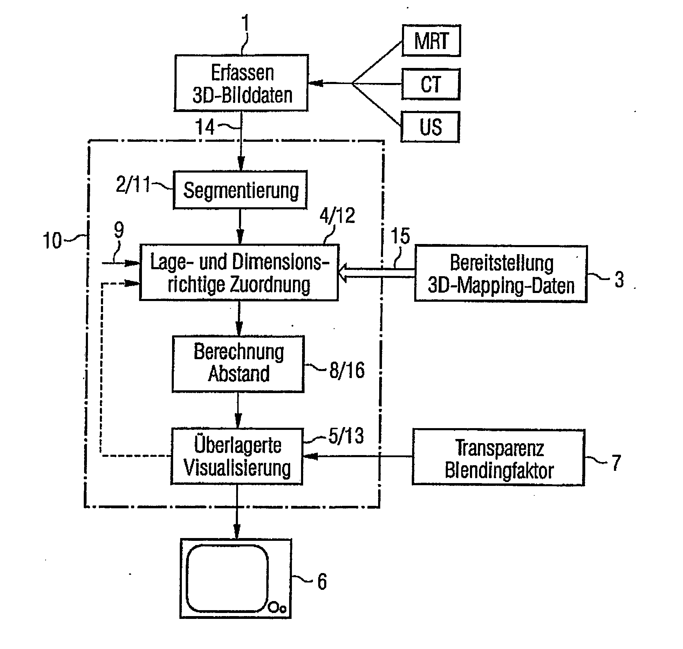

Das vorliegende Verfahren sowie die zugehörige Vorrichtung werden nachfolgend in Verbindung mit der Figur nochmals näher erläutert. Die Figur zeigt hierzu die einzelnen Schritte bei der Durchführung des vorliegenden Verfahrens bzw. die einzelnen Module der zugehörigen Vorrichtung.

In einem ersten Schritt 1 erfolgt beim vorliegenden Verfahren die Erfassung der 3D-Bilddaten des zu behandelnden Bereiches, insbesondere der zu behandelnden Herzkammer. Bei der Erfassung dieser 3D-Bilddaten kann für die später durchzuführende Registrierung auch ein größerer Teil des Herzens eingeschlossen werden. Die Erfassung der 3D-Bilddaten erfolgt mit einem Verfahren der tomographischen 3D-Bildgebung, wie beispielsweise Röntgen-Computer-Tomographie, Magnetresonanz-Tomographie oder 3D-Ultraschalltechniken. Bei der Erfassung der 3D-Bilddaten ist darauf zu achten, dass diese Bilddaten jeweils für die gleiche Herzphase erfasst werden, für die auch später die elektroanatomischen 3D-Mapping-Daten bereitgestellt werden. Dies wird durch EKG-Gating der Bilderfassung sowie der Erfassung der 3D-Mapping-Daten gewährleistet, beispielsweise durch Bezugnahme auf einen Prozentwert des RR-Intervalls oder auf einen festen Zeitabstand vor oder nach dem R-Peak.

Bei der Durchführung des Verfahrens ist es wichtig, hochauflösende Bilddaten der Herzkammer zu erfassen, die während der Katheteranwendung elektroanatomisch vermessen wird. Vorzugsweise wird daher für die Erfassung der 3D-Bilddaten ein Kontrastmittel in Verbindung mit einem Testbolus oder Bolustracking eingesetzt.

Im zweiten Schritt erfolgt die Segmentierung 2 der 3D-Bilddaten zur Extrahierung des 3D-Oberflächenverlaufs von darin enthaltenen Gefäßen und Herzkammern. Diese Segmentierung ist einerseits für die spätere Darstellung des Oberflächenverlaufes dieser Objekte in der überlagerten Bilddarstellung und andererseits in einer vorteilhaften Ausgestaltung des Verfahrens für die lage- und dimensionsrichtige Zuordnung zu den 3D-Mapping-Daten erforderlich.

Die Segmentierung erfolgt im Segmentierungsmodul 11 der vorliegenden Vorrichtung 10. Dieses Segmentierungsmodul 11 erhält die erfassten 3D-Bilddaten über eine entsprechende Eingangsschnittstelle 14. In gleicher Weise werden der Vorrichtung 10 über die gleiche oder eine weitere Schnittstelle 15 die 3D-Mapping-Daten in der Regel kontinuierlich während der Dauer der elektrophysiologischen Katheteranwendung zugeführt.

Elektrophysiologische Verfahren werden in der Regel nur in einer der Herzkammern durchgeführt. Unter den Herzkammern werden im Folgenden sowohl die Ventrikel als auch die Vorhöfe verstanden. Zusätzlich zu dieser zu behandelnden Kammer ist es auch möglich, weitere Kammern oder Gefäße elektroanatomisch zu vermessen, beispielsweise den rechten Vorhof und die Vena Cava für eine Katheterablation an der Pulmonalvene im linken Vorhof. In diesem Falle umfassen die elektroanatomischen 3D-Mapping-Daten eine oder mehrere Herzkammern und/oder Herzgefäße.

Die Segmentierung der 3D-Bilddaten kann in gleicher Weise auf eine oder mehrere Herzkammern und/oder Herzgefäße, wie beispielsweise die Vena Cava oder die Pulmonalvenen, angewendet werden, um sämtliche Oberflächen zu erhalten, die durch die elektroanatomischen 3D-Mapping-Daten repräsentiert werden. Für eine Registrierung durch Oberflächenanpassung ist es allerdings nicht erforderlich, die gesamte Oberfläche bspw. der zu behandelnden Herzkammer zu segmentieren. Hierfür reicht es vielmehr aus, eine Repräsentation der Oberfläche eines interessierenden Bereiches der Kammer, beispielsweise den linken Vorhof, oder von interessierenden Bereichen von Herzgefäßen, beispielsweise der Pulmonalvenen, durch einige Oberflächenpunkte zu erhalten, mit denen die Oberflächenanpassung für die Registrierung durchgeführt werden kann. Auf der anderen Seite kann es jedoch von Vorteil sein, einen größeren Bereich, insbesondere weitere Herzkammern oder Gefäße für die Registrierung einzubeziehen.

Die Segmentierung der zu behandelnden Herzkammer - oder weiterer Kammern oder Herzgefäße - kann in Form einer 2D-Segmentierung in einzelnen Schichten erfolgen. Eine Möglichkeit besteht darin, eine vollautomatische Segmentierung aller durch das bildgebende Verfahren erhaltenen Schichten der Herzkammer durchzuführen. Alternativ hierzu können auch ein oder mehrere der Schichten interaktiv durch einen Bediener und die jeweils darauf folgenden Schichten automatisch auf Basis der Vorkenntnis der bereits segmentierten Schichten segmentiert werden. Die interaktive Segmentierung einzelner Schichten kann auch durch halbautomatische Techniken, wie beispielsweise die Technik der aktiven Konturen, unterstützt werden. Nach der Segmentierung aller Einzelschichten kann dann der 3D-Oberflächenverlauf der Herzkammer rekonstruiert werden.

Die Segmentierung kann auch als 3D-Segmentierung der zu behandelnden Herzkammer - oder weiterer Kammern oder Herzgefäße - mittels bekannter 3D-Segmentierungstechniken erfolgen. Beispiele für derartige 3D-Segmentierungstechniken sind die Schwellwerttechnik oder die Technik des Region-Growing. Falls diese vollautomatischen 3D-Segmentierungs-Algorithmen in einzelnen Fällen nicht zuverlässig arbeiten, so kann eine interaktive Eingabemöglichkeit für einen Bediener bereitgestellt werden, um beispielsweise Grauwert-Schwellwerte oder räumliche Blocker vorgeben zu können.

Der aus der Segmentierung erhaltene 3D-Oberflächenverlauf der Objekte wird dem Registrierungsmodul 12 zugeführt, in dem die lage- und dimensionsrichtige Zuordnung der 3D-Bilddaten bzw. der daraus erhaltenen Daten des 3D-Oberflächenverlaufs zu den im Schritt 3 bereitgestellten 3D-Mapping-Daten erfolgt. Die 3D-Mapping-Daten werden über einen Mapping-Katheter erhalten, der über einen in die Spitze des Katheters integrierten 6D-Positionssensor 3D-Koordinaten von Oberflächenpunkten der zu behandelnden Herzkammer liefert. Derartige Katheter sind aus dem Stand der Technik für die Katheterablation bzw. das elektroanatomische Mapping bekannt. Der Katheter wird hierbei vom Bediener über Venen oder Arterien in die jeweilige Herzkammer eingeführt. Die Führung des Katheters sowie die Erfassung der 3D-Mapping-Daten ist nicht Bestandteil des vorliegenden Verfahrens. Während der Katheterablation bzw. dem elektroanatomischen Vermessen der zu behandelnden Herzkammer werden den Mapping-Daten im Laufe der Zeit zunehmend mehr Oberflächenpunkte hinzugefügt. Diese Oberflächenpunkte werden für die Rekonstruktion der morphologischen Struktur der Kammer, d.h. für dessen Visualisierung eingesetzt. Auf diese Weise entsteht im Laufe der Zeit ein zunehmend detaillierteres Bild der zu behandelnden Herzkammer aus den elektroanatomischen 3D-Mapping-Daten.

Hierbei ist es auch möglich, vor der Durchführung der Katheterablation komplette anatomische Oberflächen anderer Kammern und Gefäße elektroanatomisch zu erfassen und zu rekonstruieren, beispielsweise des rechten Vorhofs mit der Vena Cava im Falle einer Katheterablation an der Öffnung der Pulmonalvene. Diese elektroanatomischen 3D-Mapping-Daten werden dann bereits vor der Durchführung der Katheterablation bereitgestellt und können zur späteren Registrierung beitragen.

Beim Registrierungsschritt 4 im Registrierungsmodul 12 erfolgt neben der lagerichtigen Zuordnung auch eine Anpassung der Dimensionen der 3D-Bilddaten und der 3D-Mapping-Daten. Dies ist erforderlich, um eine möglichst genaue Überlagerung der 3D-Bilddaten der Herzkammer bzw. deren Oberfläche in gleicher Position, Orientierung, Skalierung und Form mit der entsprechenden Visualisierung der Herzkammer aus den 3D-Mapping-Daten zu erreichen. Hierfür ist in der Regel eine Transformation der 3D-Bilddaten oder der 3D-Mapping-Daten erforderlich, die drei Translationsfreiheitsgrade, drei Rotationsfreiheitsgrade, drei Skalierungsfreiheitsgrade und/oder eine Anzahl. von Vektoren zur Deformation umfassen kann.

In einer ersten Ausgestaltung kann die Registrierung durch visuelle Anpassung erfolgen. Hierfür verändert ein Bediener die visualisierten Daten so lange, bis die Position, Orientierung, Skalierung und/oder Form der dargestellten Herzkammer in beiden Repräsentationen, d.h. auf Basis der 3D-Bilddaten und auf Basis der 3D-Mapping-Daten, übereinstimmt. Die visuelle Anpassung kann über eine geeignete grafische Benutzerschnittstelle 9 erfolgen.

Weiterhin können künstliche Marker für die Registrierung eingesetzt werden. So können in einer Ausgestaltung vor der Erfassung der 3D-Bilddaten die künstlichen Marker an der Brust des Patienten befestigt werden. Diese Marker bleiben während der gesamten nachfolgenden Katheteranwendung an der gleichen Position fixiert. Wenigstens drei dieser Marker sind erforderlich, um eine korrekte Registrierung, d.h. Zuordnung der Bilddaten zu den Mapping-Daten zu erreichen. Hierbei müssen Marker eingesetzt werden, die sowohl in den 3D-Bilddaten erkennbar als auch durch den Positionssensor des Mapping-Systems identifizierbar sind.

Eine weitere Ausgestaltung zur Registrierung sieht vor, globale anatomische Marker, d.h. markante natürliche Punkte des zu behandelnden Bereiches oder dessen Umgebung, für eine Registrierung zu nutzen. Diese markanten Punkte müssen in den 3D-Bilddaten identifizierbar sein und werden vorzugsweise mit dem Mapping-Katheter unter Einsatz einer fluoroskopischen Bildgebungstechnik angefahren. Derartige markante Punkte sind beispielsweise die Öffnungen der Vena Cava Superior und Inferior oder des Koronarsinus. Die markanten Punkte können dann automatisch in den 3D-Bilddaten sowie den 3D-Mapping-Daten detektiert werden, so dass eine lage- und dimensionsrichtige Zuordnung dieser Daten berechnet werden kann.

Zusätzlich kann auch eine Registrierung zwischen der Position des Mapping-Katheters und den 3D-Bilddaten über derartige Marker oder markante Punkte erfolgen. Durch diese Registrierung wird die Visualisierung der Position des Mapping-Katheters innerhalb der 3D-Bilddaten ermöglicht.

Eine weitere vorteilhafte Möglichkeit zur Registrierung der 3D-Bilddaten und der 3D-Mapping-Daten besteht in der automatischen Anpassung der auf Basis dieser Daten dargestellten Oberflächen. Nach der Segmentierung der zu behandelnden Herzkammer kann eine automatische Anpassung der extrahierten 3D-Oberflächenkontur der Herzkammer an die durch die 3D-Mapping-Daten erhaltene Oberflächenkontur der Herzkammer erfolgen. Bei Abweichungen in der Form der aus den 3D-Bilddaten und den 3D-Mapping-Daten erhaltenen Oberflächenkonturen können deformierende Anpassungsalgorithmen auf die Oberflächenkontur aus den 3D-Bilddaten oder auf die Oberflächenkontur aus den 3D-Mapping-Daten angewendet werden, um die gegenseitige Anpassung zu verbessern:

Die Oberflächenanpassung kann beispielsweise durch Minimierung von Punktabständen zwischen Oberflächenpunkten der Mapping-Daten und Oberflächenpunkten der aus den 3D-Bilddaten extrahierten 3D-Oberflächenkontur erfolgen (Punkt-zu-Punkt-Anpassung). Alternativ kann die Anpassung auch durch Minimierung von Punktabständen zwischen Oberflächenpunkten der Mapping-Daten und interpolierten Oberflächenpunkten der 3D-Bilddaten durchgeführt werden (Punkt-zu-Oberfläche-Anpassung).

Für die Durchführung der Oberflächenanpassung ist eine gute Oberflächenrepräsentation der zu behandelnden Herzkammer durch die 3D-Mapping-Daten erforderlich. Da diese Daten in der Regel jedoch über eine längere Zeitdauer gesammelt werden, d.h. zu Beginn der Katheterablation nur wenige elektroanatomische 3D-Mapping-Daten zur Verfügung stehen, wird vorzugsweise ein mehrstufiger Prozess der Registrierung durchgeführt. Hierbei erfolgt in einer anfänglichen ersten Stufe eine Registrierung durch Marker. Die Genauigkeit der Registrierung wird dann im Verlauf des Verfahrens durch Oberflächenanpassung in einem zweiten Schritt verbessert. Selbstverständlich lassen sich auch mit zunehmender Anzahl von MappingPunkten weitere Schritte der Oberflächenanpassung vornehmen, durch die gegebenenfalls eine weitere Erhöhung der Genauigkeit ermöglicht wird. Diese mehrstufige Registrierung ist von Vorteil, da die Registrierung durch Oberflächenanpassung bei entsprechend guter Oberflächenrepräsentation genauer ist als die Registrierung mittels anatomischer markanter Punkte oder künstlicher Marker, eine gute Oberflächenrepräsentation durch die Mapping-Daten jedoch erst in einem späteren Verlauf des Verfahrens erhalten wird.

In der anfänglichen ersten Stufe kann auch eine Kombination aus einer Registrierung mittels Markern und einer Registrierung mittels Oberflächenanpassung erfolgen. So kann beispielsweise eine Registrierung des linken Vorhofs durch Oberflächenanpassung einer Gefäßoberfläche z.B. der Pulmonalarterie, und zusätzlich anhand markanter anatomischer Punkte des rechten Vorhofs, z.B. des Koronarsinus oder der Öffnung der Vena Cava Inferior oder der Vena Cava Superior, erfolgen.

Eine weitere Möglichkeit zur Registrierung mittels Oberflächenanpassung besteht darin, nicht die Oberfläche der zu behandelnden Kammer für die Anpassung heranzuziehen, sondern die Oberfläche einer anderen Kammer, die bereits vor Beginn der Katheteranwendung elektroanatomisch vermessen wurde. Dies kann beispielsweise der rechte Vorhof sein, der vor einer Pulmonalvenenisolation (PVI) des linken Vorhofs vermessen wurde. Die Vermessung sollte hierbei selbstverständlich mit einer genügenden Anzahl von Oberflächenpunkten erfolgen. Die resultierenden Anpassungsparameter für diese Kammer können dann auf die während der Katheterablation erhaltenen Daten angewendet werden.

Bei den vorangehenden Ausführungsbeispielen wurde die Oberflächenanpassung als Punkt-zu-Punkt- oder Punkt-zu-Oberfläche-Anpassung realisiert. Da das Verfahren der Katheterablation an bestimmten kleineren Bereichen der zu behandelnden Kammer durchgeführt wird, liefert eine Oberflächenanpassung in diesen interessierenden Bereichen aufgrund der höheren Dichte von Mappingpunkten präzisere Ergebnisse als in anderen Bereichen der zu behandelnden Kammer. Durch eine höhere Gewichtung von Oberflächenpunkten, die innerhalb des interessierenden Bereiches liegen, beispielsweise um die Pulmonalvenen im Falle einer PVI, wird eine bessere räumliche Anpassung in diesem Bereich erreicht als in anderen Bereichen der Herzkammer. Der interessierende Bereich kann beispielsweise durch eine entsprechende Eingabe des Bedieners an einer grafischen Benutzerschnittstelle festgelegt werden.

Neben diesem anatomischen interessierenden Bereich können auch Oberflächenpunkte in der unmittelbaren Umgebung des sich bewegenden Katheters bzw. seiner bekannten Position, herangezogen werden, um eine lokale Oberflächenanpassung vorzunehmen. Die höhere Gewichtung dieser Punkte resultiert in einer besseren lokalen Anpassung um die Katheterspitze als in anderen Bereichen der zu behandelnden Kammer. Dieses Verfahren erfordert jedoch eine Registrierung in Echtzeit während der Katheteranwendung, um die Oberflächenanpassung während der Bewegung des Katheters kontinuierlich aktualisieren zu können.

Nach der Registrierung zwischen den 3D-Mapping-Daten und den 3D-Bilddaten wird in Schritt 5 im Visualisierungsmodul 13 die lage- und dimensionsrichtige Überlagerung zur Visualisierung der überlagerten Daten durchgeführt. Mit dem gestrichelten Pfeil ist in der Figur die Möglichkeit der Verfeinerung der Registrierung bzw. Überlagerung im Verlauf der Katheterablation durch einen mehrstufigen Prozess angedeutet, wie dies bereits vorangehend erläutert wurde.

Für die überlagerte Visualisierung, die beispielsweise an einem Monitor 6 erfolgen kann, lassen sich unterschiedliche Techniken einsetzen. So kann in einer Ausgestaltung die Visualisierung der 3D-Bilddaten bzw. der zu behandelnden Herzkammer mittels einer Volume-Rendering-Technik (VRT) erfolgen. Die mit der Volume-Rendering-Technik visualisierten Bilddaten können mit den vollständigen 3D-Mapping-Daten überlagert werden, die sowohl ortsaufgelöst die elektrische Aktivität als auch die momentane Position des Katheters zeigen. Die Transparenz beider Teilbilder, d.h. des Teilbildes aus den 3D-Bilddaten und des Teilbildes aus den 3D-Mapping-Daten, kann ebenso wie der Blendingfaktor der Überlagerung durch eine Bedienperson geändert werden, um geeignete Visualisierungen der Anatomie, der Elektrophysiologie oder simultan beider Eigenschaften zu erhalten. Da die Visualisierung der 3D-Mapping-Daten die Visualisierung der Position und Orientierung des Mapping-Katheters enthält, ist es auch möglich, den 3D-Bilddaten zeitweise lediglich die Darstellung der Position und Orientierung des Mapping-Katheters zu überlagern.

In einer weiteren Ausgestaltung kann die aus den 3D-Bilddaten extrahierte Oberfläche auch als oberflächenschattierte Darstellung oder nach einer Triangulation als Polygonnetz visualisiert werden. Das Polygonnetz wird zusammen mit den 3D-Mapping-Daten dargestellt, um die durch das Polygonnetz repräsentierte Anatomie und die durch die 3D-Mapping-Daten repräsentierte Elektrophysiologie gleichzeitig zu visualisieren. Auch in diesem Fall ist es möglich, zeitweise lediglich die Position und Orientierung des Mapping-Katheters zusammen mit dem die Oberfläche repräsentierenden Polygonnetz darzustellen.

In einer weiteren Ausgestaltung lässt sich aus den erfassten Daten auch eine endoskopische Perspektive berechnen und durch Überlagerung der anatomischen 3D-Bilddaten und elektrophysiologischen 3D-Mapping-Daten visualisieren. Durch diese endoskopische Perspektive, aus dem Blickwinkel der Spitze des Katheters, kann der Katheter durch den Bediener ebenfalls an die entsprechenden anatomischen oder elektrophysiologischen Positionen, beispielsweise die Öffnung der Pulmonalvene, geführt werden.

Weiterhin lassen sich die erfassten Daten auch einsetzen, um den Abstand der Katheterspitze zu vorbestimmbaren Bereichen zu visualisieren. Da bei der Registrierung zwischen den 3D-Mapping-Daten und den 3D-Bilddaten oder bei der Registrierung zwischen der Position des Mapping-Katheters und den 3D-Bilddaten eine räumliche Beziehung zwischen dem Mapping-Katheter und den 3D-Bilddaten erhalten wird, kann jederzeit der Abstand der Spitze des Katheters zu vorgebbaren Bildpunkten des 3D-Bildes berechnet werden. Durch diese Registrierung ist es möglich, den Mapping-Katheter innerhalb der Darstellung der 3D-Bilddaten - auch ohne die Anzeige der elektrophysiologischen Daten - anzuzeigen und gleichzeitig den genannten Abstand anzugeben. So kann beispielsweise der Abstand der Katheterspitze zum Zielgewebe in der Darstellung in Echtzeit visualisiert werden. Die Visualisierung kann beispielsweise durch farbliche Darstellung des Katheters mit einer Farbkodierung des Abstandes erfolgen. Diese Möglichkeit der Katheterdarstellung kann für die Planung und Kontrolle von Ablationsprozessen eingesetzt werden. Weiterhin ist es aufgrund der Registrierung zwischen dem Mapping-Katheter und den 3D-Bilddaten auch möglich, die Position von verödeten Stellen zusammen mit den Bilddaten abzuspeichern. Die abgespeicherten Positionen können für Dokumentationszwecke sowie für die Planung und Kontrolle von nachfolgenden Ablationsprozessen verwendet werden.

## Patentansprüche

1. Verfahren zur visuellen Unterstützung einer elektrophysiologischen Katheteranwendung im Herzen, bei dem während der Durchführung der Katheteranwendung bereit gestellte elektroanatomische 3D-Mapping-Daten eines zu behandelnden Bereiches des Herzens visualisiert werden, mit einem Verfahren der tomographischen 3D-Bildgebung vor der Durchführung der Katheteranwendung 3D-Bilddaten des zu behandelnden Bereiches erfasst werden, aus den 3D-Bilddaten durch Segmentierung ein 3D-oberflächenverlauf von Objekten in dem zu behandelnden Bereich extrahiert wird, und die elektroanatomischen 3D-Mapping-Daten und zumindest den 3D-Oberflächenverlauf bildende 3D-Bilddaten durch Registrierung läge- und dimensionsrichtig zugeordnet und einander überlagert visualisiert werden, wobei zumindest in einem Stadium der Registrierung die lage- und dimensionsrichtige Zuordnung automatisch durch Oberflächenanpassung erfolgt, indem der 3D-Oberflächenverlauf aus den 3D-Bilddaten mit einem 3D-Oberflächenverlauf aus den 3D-Mapping-Daten zumindest annähernd in Übereinstimmung gebracht wird.

2. Verfahren nach Anspruch 1,
bei dem die 3D-Bilddaten des zu behandelnden Bereiches mit einem Verfahren der Röntgen-Computertomographie oder der Magnetresonanztomographie erfasst werden.

3. Verfahren nach Anspruch 1,
bei dem die 3D-Bilddaten des zu behandelnden Bereiches mit einem 3D-Ultraschallverfahren erfasst werden.

4. Verfahren nach einem der Ansprüche 1 bis 3,
bei dem die lage- und dimensionsrichtige Zuordnung automatisch in einem ersten Stadium während der Durchführung der Katheteranwendung anhand markanter anatomischer Punkte oder künstlicher Marker erfolgt und in einem späteren zweiten Stadium durch die Oberflächenanpassung verfeinert wird.

5. Verfahren nach einem der Ansprüche 1 bis 4,
bei dem die Visualisierung der 3D-Bilddaten über eine Volumen-Rendering-Technik erfolgt.

6. Verfahren nach einem der Ansprüche 1 bis 4,
bei dem die Visualisierung des 3D-Oberflächenverlaufs aus den 3D-Bilddaten als Polygonnetz erfolgt.

7. Verfahren nach einem der Ansprüche 1 bis 6,
bei dem die Überlagerung mit einstellbarer Transparenz und einstellbarem Blendingfaktor erfolgt.

8. Verfahren nach einem der Ansprüche 1 bis 7,
bei dem eine Registrierung zwischen einem bei der Katheteranwendung eingesetzten Katheter und den 3D-Bilddaten erfolgt und zumindest ein Teil des Katheters in Echtzeit in der Darstellung der zumindest den 3D-Oberflächenverlauf bildenden 3D-Bilddaten visualisiert wird.

9. Verfahren nach Anspruch 8,
bei dem die Visualisierung des mindestens einen Teils des Katheters zeitweise ohne Überlagerung der 3D-Mapping-Daten erfolgt.

10. Verfahren nach Anspruch 8 oder 9,
bei dem jeweils ein momentaner Abstand einer Spitze des Katheters zu einem vorgebbaren Bildpunkt der 3D-Bilddaten berechnet und der Abstand in der Visualisierung kodiert dargestellt wird.

11. Verfahren nach Anspruch 10,
bei dem der Abstand durch farbliche Kodierung der Visualisierung des Katheters dargestellt wird.
